# FASCICULE DE BREVET EUROPEEN

(11) **EP 2 961 451 B1**
(45) Date de publication et mention de la délivrance du brevet: **21.12.2016**
(21) Numéro de dépôt: 14713177.5
(22) Date de dépôt: 25.02.2014
(51) Int. Cl.: A61M 5/34, A61M 5/31, A61M 5/32

(54) **DISPOSITIF DE PROTECTION D'AIGUILLE**
NADELSCHUTZVORRICHTUNG
NEEDLE PROTECTION DEVICE

(30) Priorité: 01.03.2013 FR 1351849
(43) Date de publication de la demande: 06.01.2016
(73) Titulaire: Aptar Stelmi SAS, 93420 Villepinte (FR)
(72) Inventeur: FOURNIER, Arnaud, 75007 Paris (FR); FOURNIER, Ghislain, 17000 La Rochelle (FR); SWAL, Mickaël, 77124 Chauconin Neufmontiers (FR)
(74) Mandataire: CAPRI
(86) Numéro de dépôt international: PCT/FR2014/050395
(87) Numéro de publication internationale: WO 2014/131981

(56) Documents cités:
- WO-A1-2012/012601
- FR-A1- 2 803 530
- US-A- 4 829 277
- US-A- 4 966 591
- US-A- 6 156 012
- US-A1- 2009 171 311

## Description

La présente invention concerne un dispositif d'injection comportant un dispositif de protection d'aiguille.

Les dispositifs de protection d'aiguille, aussi appelés protèges-aiguille, sont bien connus. Il en existe différents types, dont les protèges-aiguille rigides, comportant un corps interne en matériau souple et un corps externe en matériau rigide. Le corps interne assure l'étanchéité à la fois avec l'orifice de l'aiguille et avec le dispositif d'injection, en général le corps de seringue, alors que le corps externe sert à fixer et maintenir le protège-aiguille sur le dispositif d'injection jusqu'à l'utilisation de ce dernier. Les documents EP 1 208 861 et FR 2 777 787 décrivent notamment des dispositifs de protection d'aiguille de ce type. Les documents US 2009/171311, WO 2012/012601, US 4 966 591, FR 2 803 530, US 4 829 277 , US 6 156 012 et US 4 950 249 décrivent d'autres dispositifs de l'état de la technique.

Ces dispositifs peuvent présenter certains inconvénients. Ainsi, il n'est pas possible d'empêcher un retrait non souhaité ou accidentel du protège-aiguille avant utilisation du dispositif d'injection associé, ce qui implique un risque de contamination de l'aiguille. De plus, en raison notamment des tolérances de fabrication, en particulier des seringues en verre, il peut être difficile dans certains cas de garantir l'étanchéité en position de stockage entre le corps interne déformable du protège-aiguille et le dispositif d'injection, en l'occurrence le corps de seringue en verre. De plus, la fabrication et l'assemblage du protège-aiguille, et notamment du corps externe sur le corps interne peut être complexe et donc coûteux. Par ailleurs, si le protège-aiguille est efficace en position de stockage, il ne permet pas de prévenir ou d'empêcher tout risque de blessure avec l'aiguille après utilisation du dispositif d'injection.

La présente invention a pour but de fournir un dispositif d'injection comportant un dispositif de protection d'aiguille qui ne reproduit pas les inconvénients susmentionnés.

La présente invention a donc pour but de fournir un tel dispositif de protection d'aiguille qui garantit l'étanchéité avant utilisation.

La présente invention a aussi pour but de fournir un tel dispositif de protection d'aiguille qui indique à l'utilisateur si l'étanchéité a été rompue avant utilisation.

La présente invention a également pour but de fournir un tel dispositif de protection d'aiguille qui soit simple et facile à fabriquer et à assembler, et fiable dans son utilisation.

La présente invention a donc pour objet un dispositif d'injection de produit fluide comportant un corps de seringue et une aiguille fixée dans une projection d'extrémité axiale du corps de seringue, ledit dispositif d'injection comportant un dispositif de protection d'aiguille, ledit dispositif de protection étant, dans une position de stockage, fixé sur ledit dispositif d'injection, ledit dispositif de protection étant amovible dudit dispositif d'injection, ledit dispositif de protection comportant un corps interne en matériau sensiblement souple ou déformable et un corps externe en matériau sensiblement rigide, ledit corps interne, en position de stockage, obturant de manière étanche l'orifice de distribution de ladite aiguille et coopérant de manière étanche avec ledit dispositif d'injection, et ledit corps externe, en position de stockage, coopérant avec ledit dispositif d'injection pour fixer ledit dispositif de protection sur ledit dispositif d'injection, ledit dispositif de protection d'aiguille comportant un système de protection contre les piqures accidentelles, ledit système de protection comportant des volets de protection formant partie intégrante dudit corps externe, lesdits volets de protection étant mobiles entre une position de recouvrement, dans laquelle ils recouvrent l'aiguille et une position ouverte, dans laquelle l'aiguille est découverte, ledit système de protection comportant en outre une bague de commande coulissant sur ledit corps externe pour, avant injection, déplacer lesdits volets de protection de leur position de recouvrement vers leur position ouverte, puis après injection, de leur position ouverte vers leur position de recouvrement.

Avantageusement, ledit corps interne comporte une projection radiale et ledit corps externe comporte un épaulement radial et un bord d'extrémité radial définissant une ouverture axiale supérieure, ledit corps interne étant inséré dans ledit corps externe à travers ladite ouverture axiale supérieure, avec ladite projection radiale en butée sur ledit épaulement radial, ledit bord d'extrémité axial dudit corps externe étant rabattu sur ledit corps interne pour fixer ledit corps interne dans ledit corps externe.

Avantageusement, ledit bord d'extrémité axial dudit corps externe est rabattu sur ledit corps interne après assemblage dudit dispositif de protection d'aiguille sur ledit dispositif d'injection.

De préférence, ledit bord d'extrémité axial dudit corps externe est rabattu sur ledit corps interne avant assemblage dudit dispositif de protection d'aiguille sur ledit dispositif d'injection.

Avantageusement, ladite projection d'extrémité axiale est définie entre une surface d'extrémité axiale du corps de seringue et une projection radiale, ledit corps externe comportant une partie de fixation fixée, notamment encliquetée, sur ladite projection d'extrémité axiale.

Avantageusement, ledit corps externe comporte une partie de protection fixée audit corps interne, ladite partie de protection reliée à ladite partie de fixation par au moins un pont de matière sécable, ledit au moins un pont de matière sécable étant cassé pour retirer ladite partie de protection et ledit corps interne dudit dispositif d'injection.

Avantageusement, la force nécessaire pour retirer ladite partie de fixation dudit dispositif d'injection est supérieure à la force nécessaire pour casser ledit au moins un pont de matière sécable.

Avantageusement, ladite bague de commande comporte au moins une partie de bord d'extrémité axial qui coopère avec lesdits volets de protection pour les déplacer de leur position de recouvrement vers leur position ouverte.

Avantageusement, lesdits volets de protection sont pivotants et reliées audit corps externe par des charnières souples.

Avantageusement, lesdits volets de protection comportent chacun au moins une patte axiale, ayant une extrémité de préférence arrondie, qui coopère avec ladite au moins une partie de bord d'extrémité axial de la bague de commande.

Avantageusement, lesdits volets de protection, en position de recouvrement, définissent au moins une fenêtre adaptée à recevoir ladite au moins une partie de bord d'extrémité axial de la bague de commande.

Avantageusement, ledit corps externe et/ou ladite bague de commande comportent des moyens de blocage pour maintenir ladite bague de commande sur ledit corps externe dans une première position, avec les volets de protection en position ouverte, et dans une seconde position, avec les volets de protection en position de recouvrement.

Avantageusement, lesdits moyens de blocages sont formés par au moins une nervure, formée sur l'un parmi le corps externe et la bague de commande, et coopérant avec au moins une rainure, formée sur l'autre parmi le corps externe et la bague de commande.

Avantageusement, ledit corps interne est réalisé en caoutchouc.

Ces caractéristiques et avantages et d'autres de la présente invention apparaîtront plus clairement à partir de la description détaillée suivante, faite en référence aux dessins joints donnés à titre d'exemples non-limitatifs, sur lesquels :
- les figures 1 et 2 sont des vues schématiques en section transversale d'un dispositif de protection d'aiguille selon une première variante de réalisation avantageuse, respectivement en position de stockage sur un dispositif d'injection et en position retirée,
- les figures 3 à 5 sont des vues schématiques en section transversale de trois autres variantes de réalisation avantageuses, en position de stockage,
- la figure 6 est une vue schématique en perspective d'encore une autre variante de réalisation, en position de stockage,
- la figure 7 est une vue schématique en section transversale du dispositif de la figure 6,
- la figure 8 est une vue similaire à celle de la figure 6, en position d'utilisation du dispositif d'injection,
- la figures 9 est une vue similaire à celle des figure 6 et 7, en position de fin d'injection, et
- la figure 10 est une vue schématique en perspective partiellement découpée, montrant le dispositif des figures 6 à 9.

La présente invention va être décrite en référence à plusieurs variantes de réalisation d'un dispositif de protection d'aiguille pour dispositif d'injection. Il est toutefois entendu que la présente invention n'est pas limitée par les modes de réalisation représentés sur les dessins.

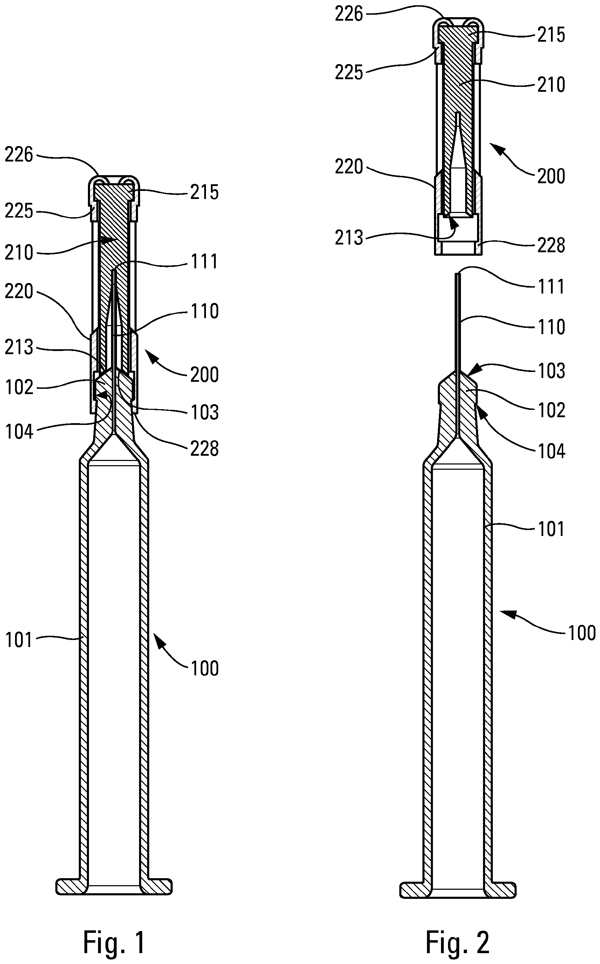

En référence aux figures 1 et 2, il est représenté un dispositif d'injection qui dans cet exemple est une seringue 100 pourvue d'un corps de seringue 101 et d'une aiguille 110 comportant un orifice de distribution 111. La partie d'extrémité axiale du corps de seringue qui fixe l'aiguille 110 comporte une projection d'extrémité axiale 102, généralement appelée boule de la seringue, définie entre la surface d'extrémité axiale 103 du corps de seringue et une projection radiale 104 prévue pour recevoir et fixer un dispositif de protection d'aiguille 200, qui sera décrit ci-après. L'aiguille a typiquement une longueur de 12,7 mm (1/2 pouce), 15,9 mm (5/8 pouce) ou 25,4 mm (1 pouce). D'autres dimensions sont aussi envisageables.

Un dispositif de protection d'aiguille 200 est prévu pour protéger et maintenir étanche ladite aiguille jusqu'à l'utilisation du dispositif d'injection. Le dispositif de protection d'aiguille 200 est fixé sur ledit dispositif d'injection 100 dans une position de stockage, et il est amovible, c'est-à-dire qu'il peut être retiré dudit dispositif d'injection au moment de l'utilisation dudit dispositif d'injection. Le dispositif de protection d'aiguille 200 comporte un corps interne 210 et un corps externe 220.

Le corps interne 210 est réalisé en matériau sensiblement souple ou déformable, tel que par exemple le polyisoprène, le styrène-butadiène (SBR), un thermoplastique élastomère (TPE) ou tout autre élastomère. D'autres matériaux sont aussi envisageables. Le corps interne 210 comporte une partie pleine qui, en position de stockage du dispositif de protection d'aiguille, reçoit l'orifice de distribution 111 de l'aiguille 110. L'extrémité axiale de l'aiguille 110 est donc enfoncée dans ledit corps interne 210 dans ladite position de stockage. Le corps interne 210 comporte aussi une projection radiale 215 formée à l'extrémité axiale distale dudit corps interne par rapport audit dispositif d'injection. De l'autre coté, à l'autre extrémité axiale, le corps interne 210 forme un manchon creux, et comporte un bord d'extrémité axial 213 proximal par rapport au dispositif d'injection dont la forme est complémentaire de la surface d'extrémité axiale 103 de la boule de la seringue 102.

Le corps externe 220 est réalisé, de préférence de manière monobloc, en un matériau sensiblement rigide, tel que par exemple le polypropylène (PP), le polystyrène (PS), le polyoxyméthylène (POM) ou le polybutylène téréphtalate (PBT). D'autres matériaux sont aussi envisageables. Le corps externe 220 comporte une partie de fixation 228 qui est adaptée à coopérer avec le corps de seringue 101, notamment avec la boule de la seringue 102, et plus particulièrement avec l'épaulement radial 104, pour fixer, notamment par encliquetage, ledit dispositif de protection d'aiguille 200 sur ledit dispositif d'injection 100. Le corps externe 220 comporte de l'autre coté, à savoir du coté distal par rapport au dispositif d'injection, un épaulement radial 225 et un bord d'extrémité axial 226 qui définit une ouverture axiale supérieure. Ledit bord d'extrémité axial est rabattable vers l'intérieur, comme cela sera expliqué ultérieurement.

Le dispositif de protection d'aiguille 200 est avantageusement réalisé par moulage des corps interne 210 et externe 220, puis assemblage du corps interne dans le corps externe. De préférence, le corps interne est inséré dans le corps externe à travers l'ouverture supérieure, jusqu'à ce que la projection radiale 215 du corps interne 210 vienne en appui sur l'épaulement radial 225 du corps externe 220. Le bord d'extrémité axial 226 du corps externe 220 est alors rabattu pour maintenir fixement le corps interne 210 dans le corps externe 220. Ce rabattement est réalisé de préférence avant assemblage du dispositif de protection d'aiguille 200 sur le dispositif d'injection 100, mais il pourrait aussi être réalisé après cet assemblage. Le rabattement du bord d'extrémité axial 226 est de préférence réalisé à chaud, typiquement entre à 100°C et 200 °C en fonction du matériau du corps externe.

En position de stockage, l'étanchéité avec l'aiguille 110 est réalisée par l'enfoncement de l'orifice de distribution 111 de l'aiguille 110 dans le matériau souple ou déformable du corps interne 210. L'étanchéité avec le dispositif d'injection est avantageusement réalisée par contact entre le bord d'extrémité axial 213 du corps interne et la surface d'extrémité axiale 103 de la boule de la seringue 102. Cette étanchéité est renforcée par la compression du corps interne 210 sur la surface d'extrémité axiale 103 de la boule de la seringue 102, compression générée notamment par la position de l'épaulement radial 225 et la longueur du corps interne 210 et/ou la pression du corps externe 220 sur le corps interne 210 au niveau du rabattement 226.

L'étanchéité en position de stockage avec le dispositif d'injection, notamment le corps de seringue, est avantageusement réalisée uniquement par contact dudit bord d'extrémité axial proximal 213 du corps interne 210 avec la surface d'extrémité axiale 103 de la boule de la seringue 102. Dans ce cas, ledit corps interne est de préférence réalisé en caoutchouc, qui présente des propriétés bien meilleures par rapport par exemple à un TPE (thermoplastique élastomère). Ainsi, le caoutchouc présente notamment les propriétés suivantes : une dureté supérieure à 60 Shore A, une élasticité supérieure à 10MPa, une déformation rémanente à la compression inférieure à 25%, une densité supérieure à 1, notamment supérieure à 1,3. De plus, le caoutchouc ne présente pas ou que très peu de variations dimensionnelles en cas de trairement à des températures élevées, par exemple environ 120°C pour la stérilisation vapeur. Au contraire, les TPE peuvent à cette occasion présenter des retraits, c'est-à-dire des diminutions dimensionnelles pouvant aller jusqu'à 3%. Dans le cas d'un corps interne d'un dispositif de protection d'aiguille, un tel retrait peut signifier la perte de l'étanchéité en position de stockage.

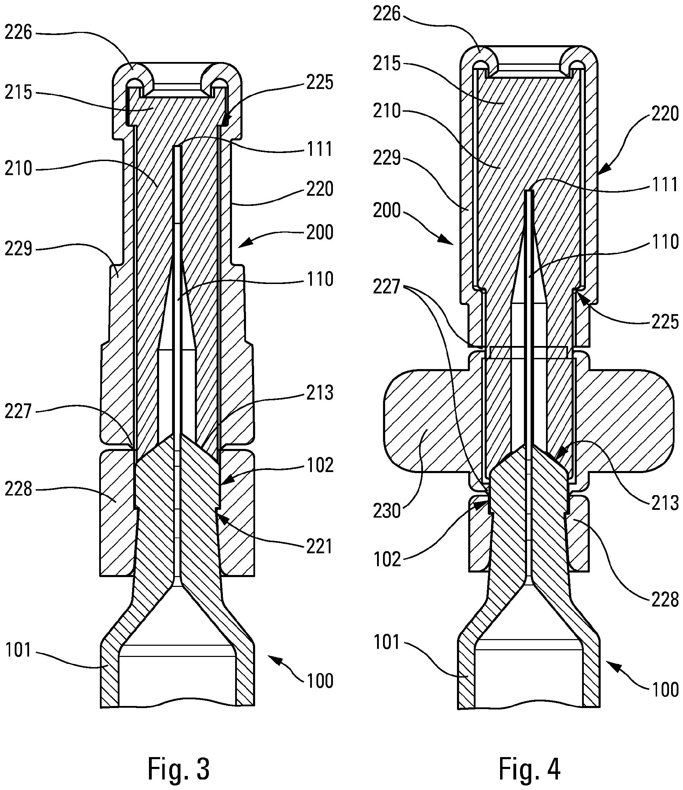

Les figures 3 à 5 montrent des variantes de réalisation avantageuses incorporant un témoin de premier usage.

Dans ces variantes, le corps externe 220 comporte une partie de protection 229 qui est reliée à la partie de fixation 228 par au moins un pont de matière sécable 227. Ainsi, le corps externe 220 est réalisé d'une seule pièce monobloc, et lors du retrait du dispositif de protection d'aiguille, ledit au moins un pont de matière sécable 227 est cassé, ce qui forme un témoin de premier usage. Pour pouvoir retirer le dispositif de protection d'aiguille, il faut casser le ou les pont(s) de matière sécable(s), et une fois cassés, il n'est plus possible de les remettre à l'état initial. L'utilisateur qui voit les ponts de matières intacts sait donc que le dispositif de protection d'aiguille n'a pas été retiré. Si par contre les ponts de matières sont cassés, il sait qu'il y a un risque de perte d'étanchéité et donc de contamination. Bien entendu, la force nécessaire pour démonter la partie de fixation 228 du dispositif d'injection 100 doit être supérieure à la force nécessaire pour casser le ou les pont(s) de matière. Ainsi, on s'assure que ce seront toujours d'abord les ponts de matière qui casseront, et il ne sera alors pas possible de retirer le dispositif de protection d'aiguille sans casser lesdits ponts de matière.

La partie de fixation 228 est fixée sur le dispositif d'injection, notamment encliquetée sur la boule de la seringue, comme décrit précédemment. La partie de protection 229 est fixée audit corps interne 210, notamment par coincement de la projection radiale 215 du corps interne 210 entre l'épaulement radial 225 et le bord d'extrémité axial rabattu 226.

La figure 3 montre une variante de réalisation dans laquelle les ponts de matière 227 sont cassés en tirant axialement sur la partie de protection 229. Lorsque la force est suffisante, les ponts de matière 227 se cassent, et la partie de protection ensemble avec le corps interne 210 peuvent être retirés du dispositif d'injection 100, alors que la partie de fixation 228 reste fixée à la boule de la seringue.

Sur la figure 4, le ou les pont(s) de matière 227 sont cassés en tirant latéralement sur une languette 230, qui est fixée d'une part à ladite partie de fixation 228 par au moins un pont de matière 227 et d'autre part à ladite partie de protection 229 par au moins un pont de matière 227.

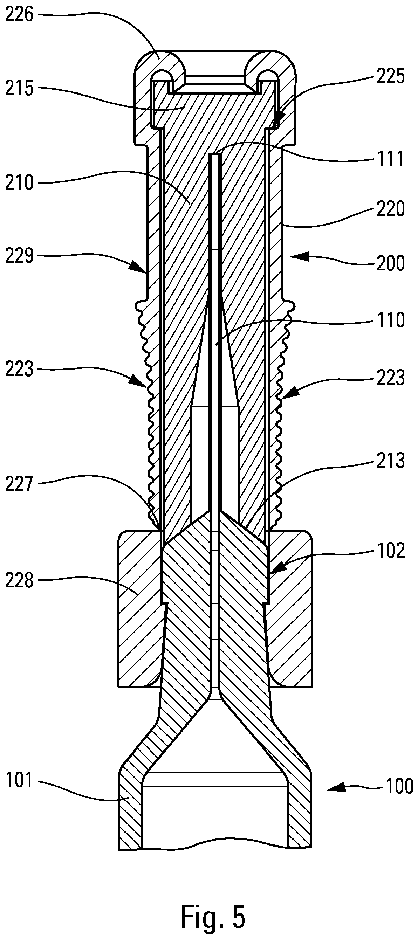

Sur la figure 5, la partie de protection 229 du corps externe 220 comporte au moins une zone de déformation manuelle 223, la rupture du ou des pont(s) de matière 227 étant réalisée par pincement de ladite au moins une zone de déformation manuelle 223. Avantageusement, deux zones de déformation manuelle 223 sont prévues en étant diamétralement opposée l'une à l'autre, chacune desdites zones 223 étant reliée à un pont de matière 227 qui se casse lorsque la zone correspondante est pincée ou autrement déformée manuellement.

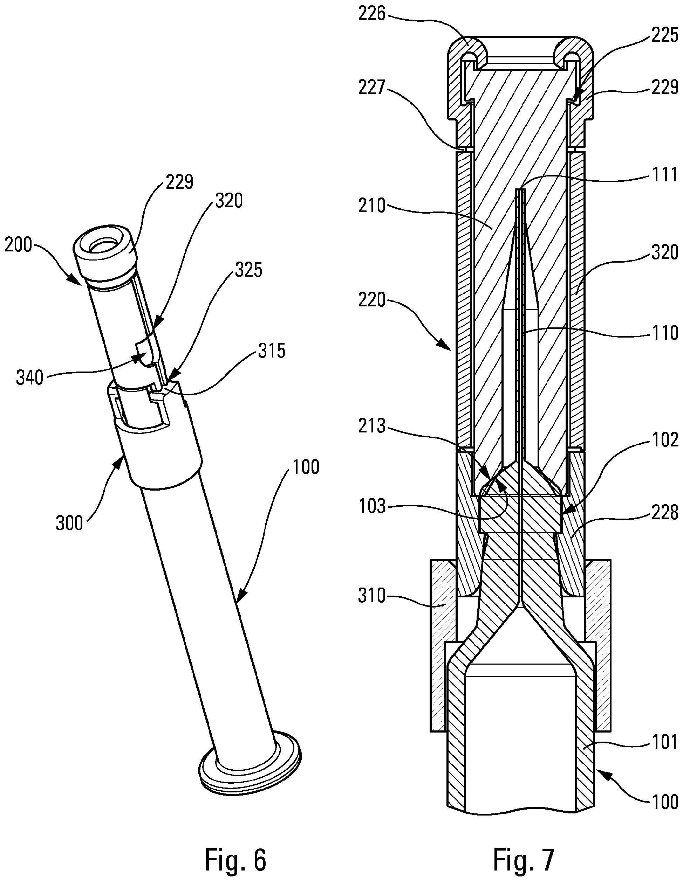

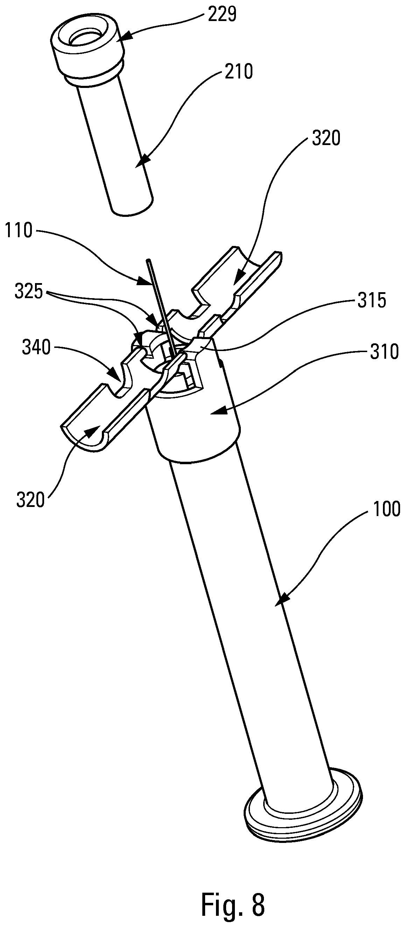

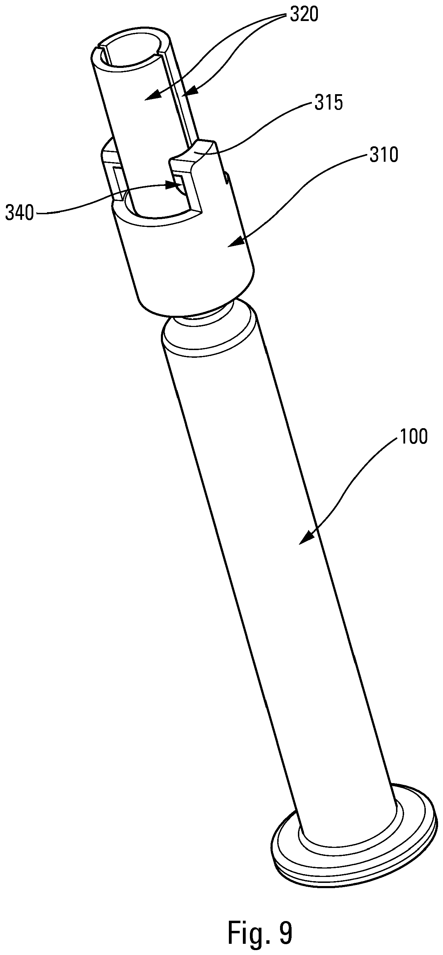

Les figures 6 à 9 illustrent une autre variante de réalisation, dans laquelle le dispositif de protection d'aiguille 200 comporte en outre un système de protection 300 contre les piqures accidentelles, notamment après utilisation du dispositif d'injection 100.

Dans cette variante, le système de protection 300 comporte une bague de commande 310, réalisée de préférence en plastique rigide comme le corps externe 220 du dispositif de protection d'aiguille 200. Cette bague de commande 310 est montée coulissante sur ledit corps externe 220.

Le corps externe 220 se décompose avantageusement en deux parties. Une partie de protection 229 permettant le retrait du corps interne 210, et une partie de fixation 228 qui se fixe sur le dispositif d'injection 100 et qui comporte en outre des volets de protection 320. La partie de protection 229 est reliée à la partie de fixation 228, notamment auxdits volets de protection 320, par des ponts de matière sécables 227. La bague de commande 310 est montée coulissante autour de ladite partie de fixation 228.

La bague de commande 310 comporte au moins une partie de bord d'extrémité axial 315, de préférence deux, qui coopère(nt) avec lesdits volets de protection 320 comme cela sera décrit ci-après.

Les volets de protection 320 sont pivotants et reliés à ladite partie de fixation 228 par des charnières souples. Ils comportent des pattes axiales 325, ayant des extrémités de préférence arrondies, qui coopèrent avec lesdites parties de bord d'extrémité axial 315 de la bague de commande 310. Les volets de protection 325, en position fermée ou de recouvrement, définissent des fenêtres 340 visibles notamment sur la figure 6.

Pour réaliser l'injection le patient ou le personnel de santé doit casser lesdits ponts de matière 227, retirer la partie de protection 229 ensemble avec le corps interne 210, et faire coulisser la bague de commande 310 dans une première position, ce qui ouvrira les volets de protection 320, et permettra de réaliser l'injection, l'aiguille 110 étant découverte.

Après injection, afin de réduire le risque de piqure accidentelle, le patient ou le personnel de santé poussera la bague de commande 310 dans une deuxième position, ce qui entrainera le recouvrement de l'aiguille 110 par les volets de protection 320.

La découverture de l'aiguille 110 se fait en deux étapes. Une première étape consiste à retirer le corps interne 210 du protège aiguille, la seconde étape réside dans l'ouverture des volets de protection 320.

Le retrait du corps interne 210 est réalisé par le retrait de la partie de protection 229 qui est possible après la cassure des ponts de matière sécables 227.

L'ouverture des volets de protection 320 se réalise en faisant coulisser la bague de commande 310 vers l'orifice de distribution 111 de l'aiguille 110. Cette remontée (dans la position des figures 6 à 9) engendre une poussée de la bague de commande 310 sur les volets de protection 320, les parties de bord d'extrémité axial 315 de la bague de commande 310 poussant sur les extrémités des pattes axiales 325 des volets de protection 320. Cette poussée provoque un pivotement des volets de protection 320 autour de leurs charnières souples, découvrant ainsi l'aiguille 110.

Après l'injection, la mise en sécurité de l'aiguille 110 se fait par une deuxième poussée de la bague de commande 310 vers l'extrémité de l'aiguille qui comporte l'orifice de distribution 111. Cette deuxième poussée doit donc être suffisante pour surmonter lesdits premiers moyens de blocage.

Cette deuxième poussée implique la libération des pattes axiales 325, permettant à ladite bague de commande 310 de pousser les volets de protection 320 pour les refermer.

Les fenêtres 340 permettent le passage de la bague de commande 310, et notamment des parties de bord d'extrémité axial 315, lors du recouvrement de l'aiguille 110 par la fermeture des volets de protection 320.

Cette position avec l'aiguille découverte est avantageusement maintenue par des premiers moyens de blocage, par exemple une première rainure 381 de la bague de commande 310 qui coopère avec une première nervure 281 de la partie de fixation 228.

Cette position est avantageusement maintenue par des seconds moyens de blocage, par exemple une seconde rainure 382 de la bague de commande 310 et/ou une seconde nervure 282 de la partie de fixation 228.

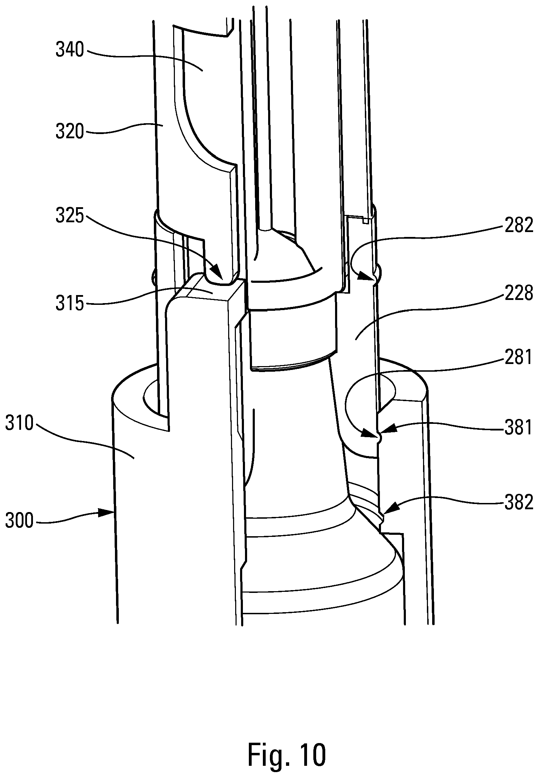

Des moyens de blocage de la bague de commande 310 par rapport audit corps externe 220 sont avantageusement prévus sur ladite bague de commande et/ou sur ledit corps externe, notamment sa partie de fixation 228. Ces moyens de blocage peuvent être réalisés de diverses manières. Par exemple, comme représenté sur la figure 10, la bague de commande 310 peut comporter deux rainures 381, 382 décalées axialement, et la partie de fixation 228 du corps externe 220 peut comporter deux nervures 281, 282, également décalées axialement. Avant actionnement, la première rainure 381 coopère avec la première nervure 281. Lorsque l'utilisateur veut utiliser le dispositif, il pousse sur la bague de commande 310 avec une force suffisante pour surmonter ces moyens de blocage et faire coulisser la bague de commande sur ledit corps externe 220, pour ouvrir les volets de protection 320, comme expliqué précédemment. Dans la position ouverte des volets, la seconde rainure 382 de la bague de commande va coopérer avec la première nervure 381 du corps externe, pour maintenir la bague de commande dans cette position d'utilisation. Après injection, pour refermer les volets 320, l'utilisateur doit à nouveau exercer une force suffisante pour surmonter les moyens de blocage, et amener la bague de commande 310 vers la position de recouvrement des volets de protection, dans laquelle la première rainure 381 de la bague de commande va coopérer avec une seconde nervure 282 du corps externe.

Bien entendu, les moyens de blocage peuvent être réalisés de différentes manières. Ainsi, une seule rainure de la bague de commande peut coopérer avec deux ou trois nervures de la partie de fixation. En variante, deux ou trois rainures de la bague de commande pourraient coopérer avec une seule nervure de la partie de fixation. Eventuellement, on peut prévoir deux nervures et deux rainures décalées de manière identique, de sorte qu'en position d'ouverture des volets, il y a un double encliquetage des deux rainures sur les deux nervures.

Bien entendu, ces moyens de blocage pourraient aussi être inversés, avec une ou deux rainures sur la partie de fixation et une ou deux nervures sur la bague de commande. D'autres mises en oeuvre sont aussi possibles.

L'exemple des figures 6 à 9 combine un système de protection 300 avec un témoin de premier usage, mais il est tout à fait possible de réaliser le système de protection 300 indépendamment du témoin de premier usage.

Bien entendu, l'invention n'est pas limitée aux exemples de réalisation représentés sur les dessins, et la portée de l'invention est au contraire définie par les revendications annexées.

## Revendications

1. Dispositif d'injection de produit fluide (100) comportant un corps de seringue (101) et une aiguille (110) fixée dans une projection d'extrémité axiale (102) du corps de seringue, ladite projection d'extrémité axiale (102) étant définie entre une surface d'extrémité axiale (103) du corps de seringue et une projection radiale (104), ledit dispositif d'injection comportant un dispositif de protection d'aiguille (200), ledit dispositif de protection étant, dans une position de stockage, fixé sur ledit dispositif d'injection, ledit dispositif de protection étant amovible dudit dispositif d'injection, ledit dispositif de protection comportant un corps interne (210) en matériau sensiblement souple ou déformable et un corps externe (220) en matériau sensiblement rigide, ledit corps interne (210), en position de stockage, obturant de manière étanche l'orifice de distribution (111) de ladite aiguille (110) et coopérant de manière étanche avec ledit dispositif d'injection (100), et ledit corps externe (220), en position de stockage, coopérant avec ledit dispositif d'injection (100) pour fixer ledit dispositif de protection (200) sur ledit dispositif d'injection (100), ledit corps externe (220) comportant une partie de fixation (228) fixée, notamment encliquetée, sur ladite projection radiale (104) de ladite projection d'extrémité axiale (102), **caractérisé en ce que** ledit dispositif de protection d'aiguille comporte un système de protection (300) contre les piqures accidentelles, ledit système de protection (300) comportant des volets de protection (320) formant partie intégrante dudit corps externe (220), lesdits volets de protection (320) étant mobiles entre une position de recouvrement, dans laquelle ils recouvrent l'aiguille (110) et une position ouverte, dans laquelle l'aiguille est découverte, ledit système de protection (300) comportant en outre une bague de commande (310) coulissant avant injection sur ledit corps externe (220) en direction de l'orifice de distribution (111) de l'aiguille (110) pour , déplacer lesdits volets de protection (320) de leur position de recouvrement vers leur position ouverte, puis coulissant après injection sur ledit corps externe (220 en direction de l'orifice de distribution (111) de l'aiguille (110) pour déplacer lesdits volets de protection (320) de leur position ouverte vers leur position de recouvrement.

2. Dispositif selon la revendication 1, dans lequel ledit corps interne (210) comporte une projection radiale (215) et ledit corps externe (220) comporte un épaulement radial (225) et un bord d'extrémité radial (226) définissant une ouverture axiale supérieure, ledit corps interne (210) étant inséré dans ledit corps externe (220) à travers ladite ouverture axiale supérieure, avec ladite projection radiale (215) en butée sur ledit épaulement radial (225), ledit bord d'extrémité axial (226) dudit corps externe (220) étant rabattu sur ledit corps interne (210) pour fixer ledit corps interne (210) dans ledit corps externe (220).

3. Dispositif selon la revendication 2, dans lequel ledit bord d'extrémité axial (226) dudit corps externe (220) est rabattu sur ledit corps interne (210) après assemblage dudit dispositif de protection d'aiguille (200) sur ledit dispositif d'injection (100).

4. Dispositif selon la revendication 2, dans lequel ledit bord d'extrémité axial (226) dudit corps externe (220) est rabattu sur ledit corps interne (210) avant assemblage dudit dispositif de protection d'aiguille (200) sur ledit dispositif d'injection (100).

5. Dispositif selon l'une quelconque des revendications précédentes, dans lequel ledit corps externe (220) comporte une partie de protection (229) fixée audit corps interne (210), ladite partie de protection (229) reliée à ladite partie de fixation (228) par au moins un pont de matière sécable (227), ledit au moins un pont de matière sécable (227) étant cassé pour retirer ladite partie de protection (229) et ledit corps interne (210) dudit dispositif d'injection (100).

6. Dispositif selon la revendication 5, dans lequel la force nécessaire pour retirer ladite partie de fixation (228) dudit dispositif d'injection (100) est supérieure à la force nécessaire pour casser ledit au moins un pont de matière sécable (227).

7. Dispositif selon l'une quelconque des revendications précédentes, dans lequel ladite bague de commande (310) comporte au moins une partie de bord d'extrémité axial (315) qui coopère avec lesdits volets de protection (320) pour les déplacer de leur position de recouvrement vers leur position ouverte.

8. Dispositif selon la revendication 7, dans lequel lesdits volets de protection (320) sont pivotants et reliées audit corps externe (220) par des charnières souples.

9. Dispositif selon la revendication 8, dans lequel lesdits volets de protection (320) comportent chacun au moins une patte axiale (325), ayant une extrémité de préférence arrondie, qui coopère avec ladite au moins une partie de bord d'extrémité axial (315) de la bague de commande (310).

10. Dispositif selon l'une quelconque des revendications 7 à 9, dans lequel lesdits volets de protection (320), en position de recouvrement, définissent au moins une fenêtre (340) adaptée à recevoir ladite au moins une partie de bord d'extrémité axial (315) de la bague de commande (310).

11. Dispositif selon l'une quelconque des revendications précédentes, dans lequel ledit corps externe (220) et/ou ladite bague de commande (310) comportent des moyens de blocage (281, 282 ; 381, 382) pour maintenir ladite bague de commande (310) sur ledit corps externe (220) dans une première position, avec les volets de protection (320) en position ouverte, et dans une seconde position, avec les volets de protection (320) en position de recouvrement.

12. Dispositif selon la revendication 11, dans lequel lesdits moyens de blocages sont formés par au moins une nervure (281, 282), formée sur l'un parmi le corps externe (220) et la bague de commande (310), et coopérant avec au moins une rainure (381, 382), formée sur l'autre parmi le corps externe (220) et la bague de commande (310).

13. Dispositif selon l'une quelconque des revendications précédentes, dans lequel ledit corps interne (210) est réalisé en caoutchouc.

## Patentansprüche

1. Injektionsvorrichtung für ein fluides Produkt (100), aufweisend einen Spritzenkörper (101) und eine Nadel (110), die in einem axialen Endvorsprung (102) des Spritzenkörpers befestigt ist, wobei der axiale Endvorsprung (102) zwischen einer axialen Endfläche (103) des Spritzenkörpers und einem radialen Vorsprung (104) definiert ist, wobei die Injektionsvorrichtung eine Nadelschutzvorrichtung (200) aufweist, wobei die Schutzvorrichtung in einer Lagerposition auf der Injektionsvorrichtung befestigt ist, wobei die Schutzvorrichtung von der Injektionsvorrichtung abnehmbar ist, wobei die Schutzvorrichtung einen internen Körper (210) aus einem im Wesentlichen flexiblen oder verformbaren Material und einen externen Körper (220) aus einem im Wesentlichen starren Material aufweist, wobei der interne Körper (210) in der Lagerposition die Ausgabeöffnung (111) der Nadel (110) dichtend verschließt und mit der Injektionsvorrichtung (100) dichtend zusammenwirkt und der externe Körper (220) in der Lagerposition mit der Injektionsvorrichtung (100) zusammenwirkt, um die Schutzvorrichtung (200) auf der Injektionsvorrichtung (100) zu befestigen, wobei der externe Körper (220) einen Befestigungsteil (228) aufweist, der auf dem radialen Vorsprung (104) des axialen Endvorsprungs (102) befestigt, insbesondere eingerastet ist,
**dadurch gekennzeichnet, dass** die Nadelschutzvorrichtung ein Schutzsystem (300) gegen unbeabsichtigte Stiche aufweist, wobei das Schutzsystem (300) Schutzklappen (320) aufweist, die einen festen Bestandteil des externen Körpers (220) bilden, wobei die Schutzklappen (320) zwischen einer Abdeckposition, in der sie die Nadel (110) bedecken, und einer geöffneten Position, in der die Nadel freigelegt ist, beweglich sind, wobei das Schutzsystem (300) des Weiteren einen Steuerring (310) aufweist, der vor der Injektion auf dem externen Körper (220) in Richtung zur Ausgabeöffnung (111) der Nadel (110) gleitet, um die Schutzklappen (320) von ihrer Abdeckposition in ihre geöffnete Position zu verschieben, und dann nach der Injektion auf dem externen Körper (220) in Richtung zur Ausgabeöffnung (111) der Nadel (110) gleitet, um die Schutzklappen (320) von ihrer geöffneten Position in ihre Abdeckposition zu verschieben.

2. Vorrichtung nach Anspruch 1, wobei der interne Körper (210) einen radialen Vorsprung (215) aufweist und der externe Körper (220) einen radialen Absatz (225) und einen radialen Endrand (226) aufweist, der eine axiale obere Öffnung definiert, wobei der interne Körper (210) über die axiale obere Öffnung in den externen Körper (220) eingefügt ist, wobei der radiale Vorsprung (215) gegen den radialen Absatz (225) anliegt, wobei der axiale Endrand (226) des externen Körpers (220) auf dem internen Körper (210) eingeschlagen ist, um den internen Körper (210) in dem externen Körper (220) zu befestigen.

3. Vorrichtung nach Anspruch 2, wobei der axiale Endrand (226) des externen Körpers (220) nach der Montage der Nadelschutzvorrichtung (200) auf der Injektionsvorrichtung (100) auf dem internen Körper (210) eingeschlagen wird.

4. Vorrichtung nach Anspruch 2, wobei der axiale Endrand (226) des externen Körpers (220) vor der Montage der Nadelschutzvorrichtung (200) auf der Injektionsvorrichtung (100) auf dem internen Körper (210) eingeschlagen wird.

5. Vorrichtung nach einem der vorhergehenden Ansprüche, wobei der externe Körper (220) einen am internen Körper (210) befestigten Schutzteil (229) aufweist, wobei der Schutzteil (229) durch mindestens eine Brücke aus einem teilbaren Material (227) mit dem Befestigungsteil (228) verbunden ist, wobei die mindestens eine Brücke aus einem teilbaren Material (227) gebrochen wird, um den Schutzteil (229) und den internen Körper (210) aus der Injektionsvorrichtung (100) herauszuziehen.

6. Vorrichtung nach Anspruch 5, wobei die Kraft, die notwendig ist, um den Befestigungsteil (228) aus der Injektionsvorrichtung (100) herauszuziehen, größer ist als die Kraft, die notwendig ist, um die mindestens eine Brücke aus einem teilbaren Material (227) zu brechen.

7. Vorrichtung nach einem der vorhergehenden Ansprüche, wobei der Steuerring (310) mindestens einen axialen Endrandteil (315) aufweist, der mit den Schutzklappen (320) zusammenwirkt, um sie von ihrer Abdeckposition in ihre geöffnete Position zu verschieben.

8. Vorrichtung nach Anspruch 7, wobei die Schutzklappen (320) schwenkbar sind und durch flexible Scharniere mit dem externen Körper (220) verbunden sind.

9. Vorrichtung nach Anspruch 8, wobei die Schutzklappen (320) jeweils mindestens einen axialen Ansatz (325) aufweisen, der ein vorzugsweise abgerundetes Ende hat, das mit dem mindestens einen axialen Endrandteil (315) des Steuerrings (310) zusammenwirkt.

10. Vorrichtung nach einem der Ansprüche 7 bis 9, wobei die Schutzklappen (320) in der Abdeckposition mindestens ein Fenster (340) definieren, das dazu geeignet ist, den mindestens einen axialen Endrandteil (315) des Steuerrings (310) aufzunehmen.

11. Vorrichtung nach einem der vorhergehenden Ansprüche, wobei der externe Körper (220) und/oder der Steuerring (310) Sperrmittel (281, 282; 381, 382) aufweist/aufweisen, um den Steuerring (310) auf dem externen Körper (220) in einer ersten Position zu halten, in der sich die Schutzklappen (320) in der geöffneten Position befinden, und in einer zweiten Position zu halten, in der sich die Schutzklappen (320) in der Abdeckposition befinden.

12. Vorrichtung nach Anspruch 11, wobei die Sperrmittel durch mindestens eine Rippe (281, 282) gebildet sind, die auf einem von dem externen Körper (220) und dem Steuerring (310) gebildet ist, und die mit mindestens einer Nut (381, 382) zusammenwirkt, die auf dem anderen von dem externen Körper (220) und dem Steuerring (310) gebildet ist.

13. Vorrichtung nach einem der vorhergehenden Ansprüche, wobei der interne Körper (210) aus Kautschuk gefertigt ist.

## Claims

1. A fluid injection device (100) comprising a syringe body (101) and a needle (110) that is fastened in an axial end projection (102) of the syringe body, said axial end projection (102) being defined between an axial end surface (103) of the syringe body and a radial projection (104), said injection device further comprising a needle protection device (200), said protection device being fastened on said injection device in a stored position, said protection device being removable from said injection device, said protection device comprising an inner body (210) that is made of material that is substantially flexible or deformable, and an outer body (220) that is made of material that is substantially rigid, in the stored position said inner body (210) closing the dispenser orifice (111) of said needle (110) in leaktight manner, and co-operating with said injection device (100) in leaktight manner, and in the stored position said outer body (220) co-operating with said injection device (100) so as to fasten said protection device (200) on said injection device (100), said outer body (220) including a fastener portion (228) that is fastened, in particular snap-fastened, on said radial projection (104) of said axial end projection (102), said injection device being **characterized in that** said needle protection device includes a protection system (300) for protecting against accidental pricking, said protection system (300) comprising protective flaps (320) that form an integral part of said outer body (220), said protective flaps (320) being movable between a covering position in which they cover the needle (110), and an open position in which the needle is uncovered, said protection system (300) further comprising a control ring (310) that, before injection, slides over said outer body (220) towards the dispenser orifice (111) of the needle (110) so as to move said protective flaps (320) from their covering position to their open position, and, after injection, slides over said outer body (220) towards the dispenser orifice (111) of the needle (110) so as to move said protective flaps (320) from their open position to their covering position.

2. A device according to claim 1, wherein said inner body (210) includes a radial projection (215), and said outer body (220) includes a radial shoulder (225) and a radial end edge (226) that defines a top axial opening, said inner body (210) being inserted in said outer body (220) through said top axial opening, with said radial projection (215) in abutment against said radial shoulder (225), said axial end edge (226) of said outer body (220) being folded down on said inner body (210) so as to fasten said inner body (210) in said outer body (220).

3. A device according to claim 2, wherein said axial end edge (226) of said outer body (220) is folded down on said inner body (210) after assembling said needle protection device (200) on said injection device (100).

4. A device according to claim 2, wherein said axial end edge (226) of said outer body (220) is folded down on said inner body (210) prior to assembling said needle protection device (200) on said injection device (100).

5. A device according to any preceding claim, wherein said outer body (220) includes a protective portion (229) that is fastened to said inner body (210), said protective portion (229) is connected to said fastener portion (228) via at least one breakable bridge of material (227), said at least one breakable bridge of material (227) being broken so as to remove said protective portion (229) and said inner body (210) from said injection device (100).

6. A device according to claim 5, wherein the force required to remove said fastener portion (228) from said injection device (100) is greater than the force required to break said at least one breakable bridge of material (227).

7. A device according to any preceding claim, wherein said control ring (310) includes at least one axial end edge portion (315) that co-operates with said protective flaps (320) so as to move them from their covering position to their open position.

8. A device according to claim 7, wherein said protective flaps (320) are pivotable and are connected to said outer body (220) via flexible hinges.

9. A device according to claim 8, wherein each of said protective flaps (320) includes at least one axial tab (325), having an end that is preferably rounded, that co-operates with said at least one axial end edge portion (315) of the control ring (310).

10. A device according to any one of claims 7 to 9, wherein, in their covering position, said protective flaps (320) define at least one slot (340) that is adapted to receive said at least one axial end edge portion (315) of the control ring (310).

11. A device according to any preceding claim, wherein said outer body (220) and/or said control ring (310) include blocking means (281, 282; 381, 382) for holding said control ring (310) on said outer body (220) in a first position, with the protective flaps (320) in their open position, and in a second position, with the protective flaps (320) in their covering position.

12. A device according to claim 11, wherein said blocking means are formed by at least one rib (281, 282) that is formed on one of the outer body (220) and the control ring (310), and that co-operates with at least one groove (381, 382) that is formed on the other one of the outer body (220) and the control ring (310).

13. A device according to any preceding claim, wherein said inner body (210) is made of rubber.
